## Europäisches Patentamt
## European Patent Office
## Office européen des brevets

(11) Numéro de publication : **0 100 281**
B1

(12)
# FASCICULE DE BREVET EUROPÉEN

(45) Date de publication du fascicule du brevet :
03.12.86

(51) Int. Cl.⁴ : **G 01 N 33/00**

(21) Numéro de dépôt : 83401522.4

(22) Date de dépôt : 25.07.83

(54) Appareil d'étalonnage d'analyseurs de gaz.

(30) Priorité : 27.07.82 FR 8213104

(43) Date de publication de la demande :
08.02.84 Bulletin 84/06

(45) Mention de la délivrance du brevet :
03.12.86 Bulletin 86/49

(84) Etats contractants désignés :
BE CH DE GB IT LI NL

(56) Documents cités :
US-A- 3 689 038
US-A- 3 776 023
US-A- 3 788 545
US-A- 3 824 836
US-A- 4 036 915
ADVANCES IN INSTRUMENTATION, vol. 31, no. 3, mars 1976, pages 710-1 - 710-6, ISA AC, US J. CLEMONS et al.: "Design criteria for a portable gas phase titration calibration system"
ANALYTICAL CHEMISTRY, vol. 42, no. 8, juillet 1970, pages 871-876, US F.P. SCARINGELLI et al.: "Preparation of known concentrations of gases and vapors with permeation devices calibrated gravimetrically"
ADVANCES IN INSTRUMENTATION, vol. 29, no. 3, pages 704-1 - 704-13, ISA AC, US E.E. HUGHES et al.: "Development of standard reference materials for air quality measurement"

(73) Titulaire : **ASSOCIATION POUR LA RECHERCHE ET LE DEVELOPPEMENT DES METHODES ET PROCESSUS INDUSTRIELS (ARMINES)**
**60, Boulevard Saint-Michel**
**F-75272 Paris Cédex 06 (FR)**

(72) Inventeur : **Di Benedetto, Dominique**
**27, rue Virgile**
**F-42000 Saint-Etienne (FR)**
Inventeur : **Marchand, Jean-Claude**
**25, rue Ovide Feuillet**
**F-42000 Saint-Etienne (FR)**

(74) Mandataire : **Bruder, Michel**
**Cabinet Michel Bruder 10, rue de la Pépinière**
**F-75008 Paris (FR)**

## Description

La présente invention concerne un appareil d'étalonnage d'analyseurs de gaz, notamment d'appareils de mesure de la pollution atmosphérique.

Pour l'étude de la pollution atmosphérique (polluants gazeux, fumées, particules), on utilise des réseaux de mesure constitués de stations réparties sur un territoire donné. Dans ces stations des analyseurs de gaz mesurent en permanence les teneurs des polluants étudiés. Pour pouvoir obtenir des résultats d'analyse fiables il est nécessaire d'étalonner périodiquement les analyseurs de gaz avec les polluants gazeux qui sont contrôlés. Par ailleurs il est également nécessaire d'assurer l'intercalibrage physique des appareils d'analyse, c'est-à-dire de faire en sorte que les étalons polluant + air et l'air échantillonné soient prélevés dans les mêmes conditions dans la station de mesure.

On connaît déjà des appareils d'étalonnage qui utilisent des gaz polluants liquéfiés contenus dans des tubes de perméation généralement en « Téflon ». L'une des parois de ce tube est généralement balayée avec de l'air « zéro » c'est-à-dire ne contenant aucun polluant, ou un autre gaz tel quel l'azote, à un débit qui est constant, l'air « zéro » ou l'azote passant autour du tube de perméation. Du fait que la pression du polluant liquide contenu à l'intérieur du tube de perméation est égale à la pression de vapeur saturante et que la pression partielle du polluant dans l'air « zéro » ou l'azote passant autour du tube de perméation est nulle, il en résulte l'existence d'un gradient de pression du polluant de part et d'autre de la paroi du tube, ce gradient de pression entraînant la diffusion du polluant à travers la paroi du tube, de l'intérieur vers l'extérieur. Ce gradient de pression restant constant, le taux de diffusion à travers le tube reste constant. De tels appareils sont décrits dans les articles :

Advances in Instrumentation, Vol. 31, n° 3, 1976, p. 710/1-6 ;

Advances in Instrumentation, Vol. 29, n° 3, 1974, p. 704/1-13 ;

Un tel appareil à tube de perméation liquide présente un certain nombre d'inconvénients. En premier lieu la pression du gaz polluant croît exponentiellement avec la température, ce qui a des conséquences défavorables en ce qui concerne le maintien d'une concentration constante. Par ailleurs la pression de vapeur saturante à l'intérieur du tube de perméation liquide reste fixe, ce qui ne permet pas de faire varier le taux de perméation. En outre ces appareils ne permettent de réaliser l'étalonnage qu'avec les gaz qui sont liquéfiables dans les tubes à perméation habituels, tels que $SO_2$, $NO_2$, $H_2S$. La température de perméation à laquelle est maintenu le tube contenant le gaz polluant liquéfié est relativement basse pour ne pas créer à l'intérieur du tube des pressions trop élevées. Du fait de ces températures relativement basses, l'écart avec la température ambiante est relativement faible, ce qui a pour conséquence que les systèmes de régulation de température ne fonctionnent pas toujours dans de bonnes conditions.

On connaît par ailleurs, ainsi qu'il est décrit dans le brevet US-A-3 533 272, une chambre de perméation contenant un polluant gazeux dans lequel baigne un tube en matière perméable parcouru par un gaz vecteur, la perméation s'effectuant, dans ce cas, de l'extérieur vers l'intérieur du tube. Un tel dispositif ne permet pas, toutefois, d'ajuster d'une manière commode le taux de perméation.

La présente invention vise essentiellement à remédier à ces inconvénients en procurant un appareil de conception particulièrement simple et permettant d'effectuer un étalonnage au moyen d'un grand nombre de polluants gazeux avec une grande fiabilité.

A cet effet cet appareil d'étalonnage d'analyseurs de gaz, notamment d'appareils de mesure de la pollution atmosphérique détectant des polluants gazeux, comprenant une chambre de perméation étanche reliée à une source d'un polluant gazeux ou de plusieurs polluants gazeux compatibles chimiquement tels que $SO_2$, $NO$, $CH_4$, ...., un tube en matière perméable au gaz polluant s'étendant en travers de cette chambre, une extrémité de ce tube étant reliée à une source de gaz vecteur pur sous pression et son autre extrémité communiquant avec un mélangeur relié lui-même à une source d'air zéro de dilution, et des moyens de réglage de la température pour maintenir une température constante prédéterminée à l'intérieur de la chambre de perméation, est caractérisé en ce que la chambre de perméation étanche porte, sur ses deux faces frontales opposées, des tubes métalliques respectifs traversant ces faces frontales, s'étendant coaxialement à l'intérieur de la chambre et étant soudés, à leurs extrémités internes, à un tube intermédiaire de plus petit diamètre, ce tube étant percé, sur une partie de sa longueur, par un certain nombre de trous, et en ce que le tube de perméation est enfilé sur le tube intermédiaire perforé.

Le tube de perméation gazeuse logé dans la chambre de perméation est de préférence réalisé en « Téflon », dans le cas d'un polluant gazeux agressif, ou en silicone dans le cas d'un polluant gazeux moins agressif tel que $SO_2$, $NH_4$, ou tout autre polymère présentant des caractéristiques intéressantes pour la technique de perméation.

L'appareil d'étalonnage suivant l'invention présente divers avantages par rapport aux appareils connus. On peut en effet régler à volonté le taux de perméation en jouant sur le nombre de trous offerts à la diffusion qui sont percés dans le tube intermédiaire sur lequel est enfilé le tube de perméation.

L'appareil suivant l'invention peut également utiliser un mélange de plusieurs polluants gazeux compatibles. On peut donc faire diffuser tous les gaz tels que NO, CO, CH$_4$, etc... et non pas uniquement ceux qui sont liquéfiables dans les tubes à perméation habituels tels que SO$_2$, NO$_2$, H$_2$S.

Le dispositif de perméation peut travailler avec des mélanges de gaz compatibles chimiquement. Tous ces gaz diffusent ensemble et suivent le même destin. De ce fait une anomalie sur l'un des constituants du mélange doit se retrouver sur les autres et inversement le bon fonctionnement sur l'un des gaz assure que le fonctionnement pour les autres gaz doit être correct. On obtient ainsi une grande sécurité pour l'étalonneur car le système est « aveugle ». On contrôle toutefois la température de perméation, le débit d'air et la pression de remplissage du polluant. De plus l'utilisation de la titration en phase gazeuse dont la réaction est rapide et complète, grâce à la concentration rapide et élevée de O$_3$ et NO dans la chambre de réaction, vient renforcer encore l'interdépendance de tous les polluants.

La température de perméation est réglée de préférence à 64 °C, ce qui assure que, quel que soit le lieu d'utilisation, l'écart entre la température ambiante et celle du dispositif de perméation sera toujours suffisant pour que la régulation de température (à partir d'une source de courant continu à 12 V) fonctionne toujours dans de bonnes conditions. Ceci n'est par contre pas le cas des appareils connus utilisant des tubes à perméation liquide qui ne peuvent monter à une telle température par suite des pressions excessivement élevées atteintes par les polluants à l'intérieur du tube de perméation.

Un autre avantage de l'appareil suivant l'invention est qu'il n'exige pas de bouteille de NO puisque ce gaz est obtenu par perméation au sein du système et en outre le débit d'air « zéro » ou d'azote balayant le tube n'a pas besoin d'être régulé : il doit simplement assurer que la pression partielle du polluant à l'intérieur du tube est nulle pour que le taux de diffusion reste constant. Comme l'azote constituant le gaz vecteur contenant les polluants représente moins de 1 % du débit final, la concentration finale du ou des polluants à la sortie de l'appareil est donc indépendante du débit d'azote. Au contraire pour les appareils connus actuellement NO est obtenu directement à partir d'une bouteille à 100 ppm (par exemple) et par conséquent le débit de cette bouteille doit être soigneusement régulé afin que la concentration finale reste constante.

L'air de dilution doit être un air « zéro » ne contenant pas les polluants produits par l'appareil. Or, on ne sait pas obtenir un air ayant une teneur nulle en NO par adsorption classique sur du charbon actif ou un tamis moléculaire. Les appareils connus ne permettent donc pas de garantir un air « zéro ». Au contraire, dans l'appareil suivant l'invention, on obtient un air « zéro » en NO, NO$_2$, SO$_2$ et O$_3$ en faisant passer l'air aspiré par une pompe autour d'un générateur d'ozone : O$_3$ ainsi produit oxyde alors complètement NO en NO$_2$ et ensuite SO$_2$, O$_3$ et NO qui sont éventuellement présents dans l'air ambiant, sont alors fixés sur du charbon actif, NO ayant été préalablement transformé en NO$_2$.

Un autre avantage de l'appareil suivant l'invention est que la durée de vie d'un remplissage peut aller jusqu'à plusieurs années et que par ailleurs cet appareil est toujours prêt à fonctionner puisqu'il reste opérationnel pendant le transport (en étant alimenté à partir d'une batterie de 12 V d'un véhicule) et qu'il possède par ailleurs, avec ses batteries internes, une autonomie de trente heures.

On décrira ci-après, à titre d'exemples non limitatifs, diverses formes d'exécution de la présente invention, en référence au dessin annexé sur lequel :

La figure 1 est un schéma de principe d'un appareil d'étalonnage d'analyseurs de gaz suivant l'invention, utilisant un tube de perméation gazeuse.

La figure 2 est un schéma détaillé d'une forme d'exécution de l'appareil d'étalonnage suivant l'invention.

La figure 3 est une vue en coupe axiale d'un Té à perméation gazeuse.

La figure 4 est une vue en coupe axiale, partiellement en élévation, d'une cellule à perméation gazeuse.

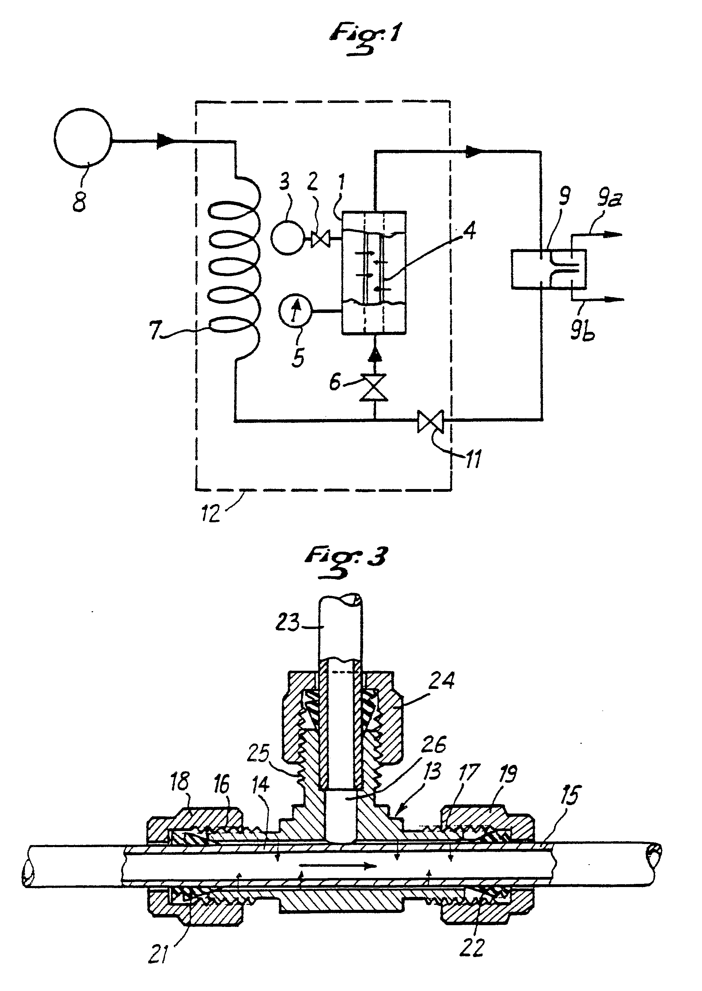

L'appareil d'étalonnage suivant l'invention, dont le principe est illustré par le schéma de la figure 1, comprend une chambre de perméation étanche 1 qui est reliée, par une vanne d'arrêt 2, à une source 3 d'au moins un gaz polluant. Cette chambre de perméation 1 est traversée de part en part par un tube de perméation 4 en une matière perméable au gaz polluant, tel que silicone ou autres polymères. La pression du gaz polluant dans la chambre 1 est contrôlée par un manomètre 5. Une extrémité du tube de perméation 4 est reliée, par l'intermédiaire d'une vanne 6 et d'un serpentin 7, à une source d'un gaz vecteur qui peut être de l'air « zéro », c'est-à-dire pur de tout gaz polluant tel que NO, NO$_2$, SO$_2$, O$_3$, ou encore de l'azote. L'autre extrémité du tube de perméation 4 est reliée à un mélangeur 9 dans lequel est également introduit directement, à travers une vanne 11, un débit d'air zéro de dilution. La chambre de perméation 1 ainsi que le serpentin 7 sont logés dans une enceinte 12 dont la température est régulée de manière à être maintenue constante et par exemple égale à 64 °C.

On voit d'après la description qui précède que le gaz vecteur (air « zéro » ou azote) s'écoule à travers le tube de perméation 4, avec un débit réglable par la vanne 6. Par ailleurs le ou les gaz polluants sont introduits dans la chambre de perméation 1, tout autour du tube de perméation 4, avec une pression partielle réglable et contrôlée au moyen du manomètre 5. A l'intérieur du tube de perméation 4 la pression partielle du gaz polluant est pratiquement nulle. Il existe donc, des deux côtés de la paroi du tube de perméation 4, un gradient de concentration. Si on maintient ce gradient constant c'est-à-dire si on maintient constante la pression partielle du gaz polluant dans la chambre 1 autour du tube 4, la vitesse de diffusion, c'est-à-dire la quantité de polluant qui traverse la paroi du tube 4 par unité de temps est constante. De ce fait on obtient une concentration constante et faible du polluant dans l'air « zéro » ou l'azote à la sortie du tube de perméation 4. La concentration finale est obtenue grâce à une dérivation sur le circuit d'air « zéro », le débit d'air « zéro » introduit directement dans le mélangeur 9 étant réglé au moyen de la vanne 11. Ce mélangeur 9 est nécessaire pour une bonne homogénéisation du mélange air + polluant qui sort vers l'utilisation par une canalisation 9a. Une autre canalisation de sortie 9b est prévue pour assurer la sortie de l'excès du mélange garantissant ainsi que la pression à l'intérieur du mélangeur 9 reste égale à la pression atmosphérique. De ce fait l'analyseur de gaz qui est ultérieurement branché sur l'appareil suivant l'invention, prélève l'étalon gazeux dans les mêmes conditions pneumatiques que les échantillons de l'atmosphère.

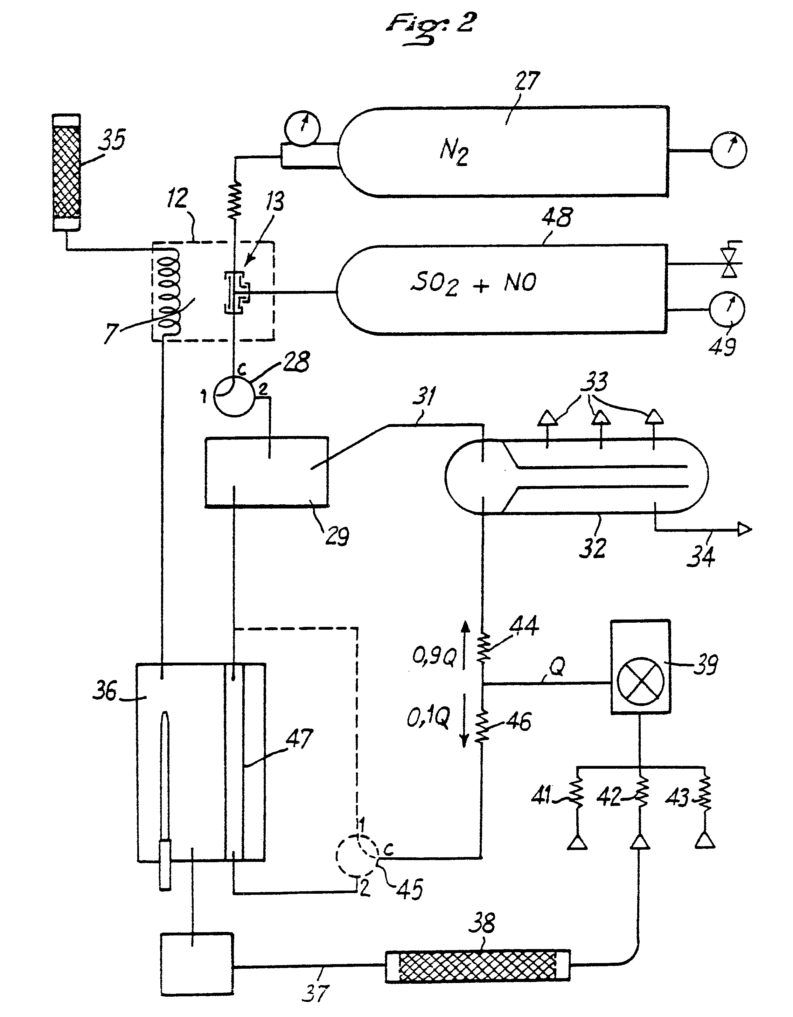

On décrira maintenant, en se référant plus particulièrement à la figure 2, une forme d'exécution particulière de l'invention, les éléments de cette figure semblables à ceux de la figure 1 étant affectés des mêmes références numériques. Dans cette forme d'exécution de l'invention la chambre de perméation est constituée par un Té 13 représenté d'une manière détaillée sur la figure 3. Ce Té comprend un corps qui est percé de part en part d'un alésage 14 à travers lequel s'étend un tube de perméation 15 réalisé par exemple en « Téflon ». Le corps du Té 13 est pourvu, aux deux extrémités de l'alésage 14, de raccords filetés 16 et 17 sur lesquels sont vissés des écrous respectifs 18 et 19 traversés par le tube de perméation 15. Ces écrous assurent le blocage du tube 15 dans les raccords 16, 17, par l'intermédiaire de bagues coniques respectives 21, 22. Ce type de raccord est connu sous le nom de « Swagelock ».

Le diamètre externe du tube de perméation 15 est inférieur au diamètre de l'alésage 14 de manière à délimiter avec la paroi de celui-ci une chambre dans laquelle est introduit le polluant gazeux. Ce polluant est fourni par une canalisation 23 qui est fixée, au moyen d'un écrou 24, sur un raccord fileté 25 entourant un conduit transversal 26 débouchant perpendiculairement dans l'alésage 14, au milieu de celui-ci.

Le tube de perméation 15 est relié, à l'une de ses extrémités, à une bouteille d'azote 27 (figure 2), tandis que son autre extrémité est connectée à une vanne à trois voies 28. Cette vanne assure la communication avec une chambre de réaction 29 qui est reliée, par une canalisation 31, à un mélangeur échantillonneur 32. Ce mélangeur 32 est pourvu de plusieurs raccords de sortie 33 destinés à être reliés aux analyseurs de gaz, ainsi que d'une canalisation 34 de sortie d'excès du mélange.

L'air atmosphérique est aspiré à travers un filtre 35 qui est relié à une extrémité du serpentin 7 logé, avec le Té de perméation 13, à l'intérieur de l'enceinte thermostatée. Le serpentin 7 est relié à son tour à un générateur d'ozone 36 dont l'intérieur communique, par une canalisation 37 sur laquelle est branché un filtre à charbon actif 38, avec l'orifice d'aspiration d'une pompe 39. Des éléments 41, 42, 43 introduisant des pertes de charge variables sont reliés à l'orifice d'entrée de la pompe 39 pour permettre de fixer trois débits différents de cette pompe à savoir 78 l/h, 135 l/h et 335 l/h par exemple. L'orifice de refoulement de la pompe 39 est relié d'une part au mélangeur échantillonneur 32, à travers un élément de perte de charge 44, et d'autre part à une vanne à trois voies 45, par l'intermédiaire d'un autre élément à perte de charge 46. Le débit Q d'air « zéro » fourni par la pompe 39 est divisé en deux à savoir un débit relativement important (par exemple 0,9 Q) dirigé directement vers le mélangeur 32 et d'autre part un débit plus faible (par exemple 0,1 Q) dirigé vers la vanne à trois voies 45. Ce débit faible peut être dirigé par cette vanne, dans une première position de celle-ci, de manière qu'il passe, en tant que gaz vecteur, à travers un tube en quartz 47 s'étendant en travers du générateur d'ozone 36 et communiquant, à son extrémité opposée, avec la chambre de réaction 29. Dans une seconde position de la vanne 45, le générateur d'ozone 36 est mis en court-circuit et le débit d'air « zéro » est dirigé directement vers la chambre de réaction 29.

Le ou les gaz polluants sont contenus dans une seule cellule de grand volume 48 qui est reliée au Té de perméation 13, par la canalisation 23. Cette cellule 48 dont la pression est contrôlée par un manomètre 49 peut contenir, par exemple, $SO_2$, $NO$, $CH_4$, $C_3H_8$ etc.

L'enceinte thermostatée 12 est maintenue à une température appropriée, de l'ordre de 64 °C. La température de l'enceinte thermostatée 12 est maintenue constante par un circuit de régulation électrique qui peut être alimenté soit à partir d'une source de courant alternatif, dans une station, soit à partir d'un accumulateur portatif incorporé dans l'appareil.

Le gaz vecteur fourni à la chambre de perméation 13 est constitué par de l'azote contenu dans la bouteille 27, l'azote étant fourni à très faible débit. Ce débit n'a pas besoin d'être régulé puisque la vitesse de perméation ne dépend pas du débit de l'azote ou de l'air « zéro », à condition que la pression partielle du polluant gazeux du gaz vecteur soit nulle et que le débit du gaz vecteur soit très faible devant le débit final obtenu au mélangeur 32. En fait le débit d'azote dans la chambre de perméation 13 est inférieur au centième du débit d'air de dilution fourni par la pompe 39 au mélangeur 32.

L'air ambiant aspiré par la pompe 39 passe dans le générateur d'ozone 36 afin d'oxyder le $NO$ de l'air ambiant en $NO_2$ : dans ces conditions $NO_2$ et l'excès d'ozone sont adsorbés sur le filtre à charbon actif 38 ainsi que $SO_2$ ou d'autres polluants adsorbables sur charbon actif.

La pompe 39 aspire ainsi l'air « zéro », dont le débit peut être réglé par les éléments de perte de charge 41, 42, 43, cet air « zéro » ne contenant aucun polluant tel que $NO$, $SO_2$, $NO_3$, $NO_2$.

Grâce au débit utilisé et au mélangeur échantillonneur 32 on peut alimenter plusieurs analyseurs de gaz en même temps, en particulier des analyseurs d'ozone et d'oxydes d'azote afin de vérifier le bon fonctionnement de la titration en phase gazeuse.

La vanne à trois voies 45 permet d'utiliser ou non la titration en phase gazeuse. .

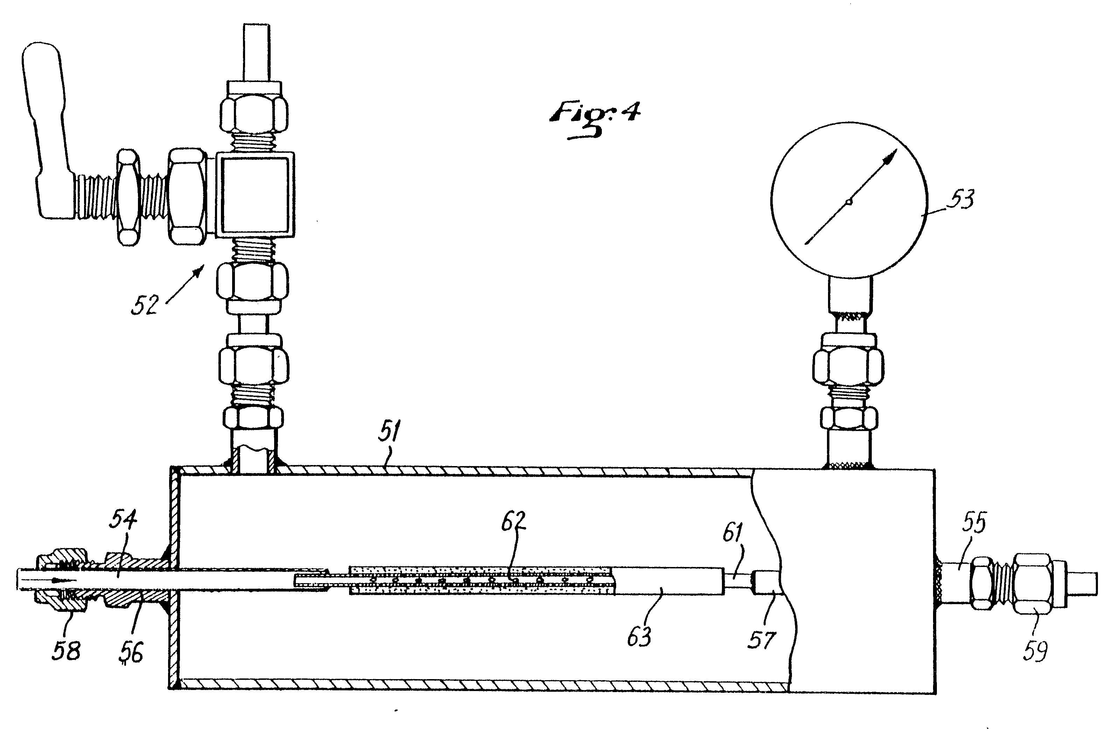

On décrira maintenant, en se référant plus particulièrement à la figure 4, une forme d'exécution non limitative d'une cellule à perméation gazeuse pouvant être utilisée notamment lorsque le polluant gazeux est constitué par de l'hydrogène sulfuré. Cette cellule comprend une chambre cylindrique 51 fermée d'une manière étanche et qui est reliée, par une vanne d'arrêt 52, à une source de polluant gazeux (hydrogène sulfuré), la pression dans la chambre 51 étant contrôlée par un manomètre 53. Cette chambre cylindrique 51 porte, sur ses deux faces frontales, des raccords d'entrée 54 et de sortie 55 traversés par des tubes métalliques respectifs 56, 57 lesquels sont bloqués au moyen d'écrous 58, 59. Les deux tubes 56, 57 s'étendent coaxialement à l'intérieur de la chambre cylindrique 51 et sont soudés, à leurs extrémités, à un tube intermédiaire 61 de plus petit diamètre, en acier inoxydable. Ce tube est percé, sur une partie de sa longueur, d'un certain nombre de trous 62. Par ailleurs un tube de perméation 63 est enfilé sur le tube perforé 61. De cette façon le polluant gazeux qui se trouve sous pression à l'intérieur de la chambre cylindrique 51, peut diffuser à travers le tube de perméation 63 qui est en silicone ou en un autre polymère, et passer à travers les trous 62 du tube interne 61 en acier inoxydable. Le polluant gazeux passe ainsi dans le gaz vecteur qui traverse, de gauche à droite, la chambre de perméation 51, à travers les tubes 56, 61, 57, le taux de perméation étant fonction du polluant gazeux ainsi que du nombre de trous perforés du tube 61. Pour régler ce taux de perméation on peut faire varier la pression du polluant gazeux dans la chambre 51 ou éventuellement le nombre de trous du tube perforé 61.

**Revendications**

1. Appareil d'étalonnage d'analyseurs de gaz, notamment d'appareils de mesure de la pollution atmosphérique détectant des polluants gazeux, comprenant une chambre de perméation étanche (51) reliée à une source (3, 48) d'un polluant gazeux ou de plusieurs polluants gazeux compatibles chimiquement tels que SO$_2$, NO, CH$_4$, ..., un tube (63) en matière perméable au gaz polluant s'étendant en travers de cette chambre, une extrémité de ce tube étant reliée à une source (8, 27) de gaz vecteur pur sous pression et son autre extrémité communiquant avec un mélangeur (32) relié lui-même à une source (35-39) d'air « zéro » de dilution, et des moyens (12) de réglage de la température pour maintenir une température constante prédéterminée à l'intérieur de la chambre de perméation (51), caractérisé en ce que la chambre de perméation étanche (51) porte, sur ses deux faces frontales opposées, des tubes métalliques respectifs (56, 57) traversant ces faces frontales, s'étendant coaxialement à l'intérieur de la chambre (51) et étant soudés, à leurs extrémités internes, à un tube intermédiaire (61) de plus petit diamètre, ce tube (61) étant percé, sur une partie de sa longueur, par un certain nombre de trous (62), et en ce que le tube de perméation (63) est enfilé sur le tube intermédiaire perforé (61).

2. Appareil suivant la revendication 1 caractérisé en ce que la source (35-39) d'air « zéro » de dilution comprend un filtre (35) relié à une extrémité d'un serpentin (7) logé, avec la chambre de perméation (13, 51), à l'intérieur d'une enceinte thermostatée (12), ce serpentin (7) est relié à son tour à un générateur d'ozone (36) dont l'intérieur communique, par une canalisation (37) sur laquelle est branché un filtre à charbon actif (38), avec l'orifice d'aspiration d'une pompe (39) dont l'orifice de refoulement est relié d'une part au mélangeur échantillonneur (32), pour lui fournir un débit d'air de dilution, et d'autre part, par l'intermédiaire d'une vanne à trois voies, à un tube en quartz (47) s'étendant en travers du générateur d'ozone (36), la vanne à trois voies (45) étant agencée de manière à mettre ou non en court-circuit le tube de perméation (47) du générateur d'ozone.

3. Appareil suivant la revendication 2, caractérisé en ce que des moyens (44, 46) sont prévus pour régler les débits d'air « zéro » respectivement vers le mélangeur échantillonneur (32) et vers le générateur d'ozone (36), de manière que le débit fourni au mélangeur échantillonneur (32) soit beaucoup plus important que le débit fourni au générateur d'ozone (36).

**Claims**

1. Calibration system for gas analyzers particularly for apparatus for measuring atmospheric pollution detecting gaseous pollutants, wherein said system comprises a tigh permeation chamber (51) connected to a source (3, 48) of a gaseous pollutant or a plurality of chemically compatible gaseous pollutants such as SO$_2$, NO, CH$_4$, ..., a tube (63) made of a material permeable to the pollutant gas extending across this chamber, one end of this tube being connected to a source (8, 27) of pure vector gas under pressure and its other end communicating with a mixer (32) itself connected to a source (35-39) of dilution zero air, and means (12) for regulating the temperature to maintain a predetermined constant temperature inside the permeation chamber, characterized in that the tight permeation chamber (51) bears, on its two front faces, respective metal tube (56, 57) passing through said front faces, said two tubes extending coaxially inside the chamber (51) and being welded, at their ends, to an intermediate tube (61) of smaller diameter, this tube (61) being pierced, over a part of its length, by a certain number of holes, and the permeation tube (63) is fitted on the perforated intermediate tube (61).

2. Calibration system for gas analyzers according to claim 1, wherein the source (35-39) of the dilution zero air comprises a filter (35) connected to one end of a coil (7) housed, with the permeation chamber (13, 51), inside a heat-regulated enclosure (12), this coil (7) is in turn connected to an ozone generator (36) whose interior communicates, via a pipe (37) on which is connected an active charcoal filter (38), with the intake orifice of a pump (39) whose delivery orifice is connected on the one hand to the sampling mixer (32), to furnish thereto a flow of dilution gas, and on the other hand, via a three-way valve, to a quartz tube (47) extending across the ozone generator (36), the three-way valve (45) being arranged so as to short-circuit, or not, the permeation tube (47) of the ozone generator.

3. Calibration system for gas analyzers according to claim 2, wherein means (44, 46) are provided for regulating the rates of flow of zero air respectively towards the sampler mixer (32) and towards the ozone generator (36), so that the rate of flow furnished to the sampling mixer (32) is much greater than the rate of flow furnished to the ozone generator (36).

**Patentansprüche**

1. Gerät zum Eichen von Gassensoren, insbesondere von Geräten zur Messung der atmosphärischen Verunreinigung, welche gasförmige Verunreinigungen ermitteln, mit einer dichten Permeationskammer (51), die mit einer Quelle (3, 48) einer gasförmigen Verunreinigung oder gasförmiger Verunreinigungen verbunden ist, die chemisch mit z. B. $SO_2$, NO, $CH_4$ ..., kompatibel ist bzw. sind, mit einem Rohr (63) aus einem für das verunreinigende Gas durchlässigen Material, das sich durch die Kammer erstreckt, wobei ein Ende dieses Rohrs mit einer Quelle (8, 27) eines reinen Vektorgases und sein anderes Ende mit einer ihrerseits mit einer Quelle (53-39) von Verdünnungsluft « Null » verbundenen Mischvorrichtung (32) verbunden ist, und mit Temperaturregelungsmitteln (12) zur Aufrechterhaltung einer konstanten, vorgegebenen Temperatur im Inneren der Permeationskammer (51), dadurch gekennzeichnet, daß die dichte Permeationskammer (51) auf ihren beiden entgegengesetzten Stirnflächen zugeordnete Metallrohre (56, 57) trägt, die diese Stirnflächen durchqueren und sich koaxial in das Innere der Kammer (51) erstrecken und mit ihren inneren Enden mit einem Zwischenrohr (61) mit kleinerm Durchmesser verschweißt sind, wobei dieses Zwischenrohr (61) über einen Teil seiner Länge mit einer gewissen Anzahl von Löchern (62) versehen ist, und daß das Permeationsrohr (63) über das perforierte Zwischenrohr (61) geschoben ist.

2. Gerät nach Anspruch 1, dadurch gekennzeichnet, daß die Quelle der Verdünnungsluft « Null » einen Filter (35) umfaßt, der mit einem Ende einer Schlange (7) verbunden ist, die mit der Permeationskammer (13, 51) in Inneren eines thermisch stabilisierten Raumes (12) angeordnet ist, wobei diese Schlange (7) ihrerseits mit einem Ozongenerator (36) verbunden ist, dessen Inneres über eine Leitung (37), in welche ein Aktivkohle-Filter (38) einbezogen ist, mit der Saugseite einer Pumpe (39) verbunden ist, deren Druckseite einerseits mit einem Probemischer (32) verbunden ist, um ihn mit Verdünnungsluft zu versorgen, und andererseits über ein Dreiwegeventil mit einem sich durch den Ozongenerator (36) erstreckenden Quarzrohr (47), wobei des Dreiwegeventil derart betätigbar ist, daß das Permeationsrohr (47) des Ozongenerators kurzgeschlossen wird oder nicht.

3. Gerät nach Anspruch 2, dadurch gekennzeichnet, daß Mittel (44, 46) vorgesehen sind, um die Lieferung der « Null »-Luft entweder zum Probemischer (32) oder zum Ozongenerator (36) zu regeln, derart, daß die dem Probemischer (32) zugeführte Menge wesentlich größer ist als die dem Ozongenerator (36) zugeführte Menge.

## *Fig.1*

## *Fig.3*

*Fig. 2*

*Fig:4*

52

51

53

54

62

61

55

58    56

63    57

59

0 100 281